(19) Europäisches Patentamt
European Patent Office
Office européen des brevets

(11) Veröffentlichungsnummer : **0 343 468 B1**

(12) # EUROPÄISCHE PATENTSCHRIFT

(45) Veröffentlichungstag der Patentschrift : **02.09.92 Patentblatt 92/36**

(51) Int. Cl.[5] : **C07C 33/02,** C07C 69/145, C07C 29/32

(21) Anmeldenummer : **89108673.8**

(22) Anmeldetag : **13.05.89**

(54) **Verfahren zur Herstellung von E7/Z9-Alkadien-1-olen und deren an der Hydroxylgruppe geschützten Derivaten.**

(30) Priorität : **21.05.88 DE 3817399**

(43) Veröffentlichungstag der Anmeldung : **29.11.89 Patentblatt 89/48**

(45) Bekanntmachung des Hinweises auf die Patenterteilung : **02.09.92 Patentblatt 92/36**

(84) Benannte Vertragsstaaten : **CH DE ES FR GB IT LI NL**

(56) Entgegenhaltungen :
**FR-A- 2 505 820**
**TETRAHEDRON LETTERS Band 26, Juni 1979, Seiten 2467-2470**
**LIEBIGS ANNALEN DER CHEMIE Band 1981. no.9, September 1981, Seiten 2117-2138**

(73) Patentinhaber : **BASF Aktiengesellschaft**
**Carl-Bosch-Strasse 38**
**W-6700 Ludwigshafen (DE)**

(72) Erfinder : **Brückner, Christiane, Dr.**
**Bad-Aussee-Strasse 55**
**W-6700 Ludwigshafen (DE)**
Erfinder : **Buschmann, Ernst, Dr.**
**Georg-Ludwig-Krebs-Strasse 10**
**W-6700 Ludwigshafen (DE)**
Erfinder : **Mackenroth, Wolfgang, Dr.**
**Seebacher Strasse 37**
**W-6702 Bad Dürkheim (DE)**
Erfinder : **Himmele, Walter, Dr.**
**Eichenweg 14**
**W-6909 Walldorf (DE)**
Erfinder : **Eckhardt, Heinz, Dr.**
**Rüdigerstrasse 7**
**W-6700 Ludwigshafen (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 343 468 B1

## Beschreibung

Die vorliegenden Erfindung betrifft die Herstellung von E7/Z9-Alkadien-1-olen der allgemeinen Formel I

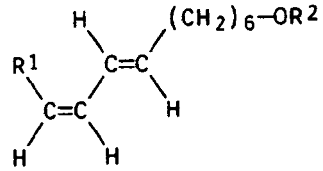

I,

in der die Reste $R^1$ $C_1$-$C_8$-Alkyl und $R^2$ Wasserstoff oder eine basenstabile Schutzgruppe bedeuten.

Insbesondere betrifft die Erfindung die Herstellung von E7/Z9-Dodecadienylacetat ($R^1 = C_2H_5$ und $R^2 = COCH_3$), dem Sexualpheromon des bekreuzten Traubenwicklers, Lobesia botrana. Durch den Einsatz dieses Pheromons, das eine attraktive und stark stimulierende Wirkung auf die männlichen Tiere ausübt, kann dieser Schädling, der als bedeutendstes Schadinsekt im Weinbau in Mittel- und vorweigend Südeuropa anzusehen ist, wirksam kontrolliert werden (siehe z.B. Röhrich et al, rev. Zool. Agric. Pathol. Veg. 76, 25, 1977).

Zur Synthese dieses demzufolge wirtschaftlich relevanten Pheromons wurden bisher verschiedene Methoden beschrieben, die sich in der Mehrzahl Kupplungsreaktionen von Acetylenderivaten bedienen (Labowitz et al., Tetrahedron Lett. 1975, 4209-4212; Negishi et al., Tetrahedron Lett. 1977, 411-414; Descoins et al., Bull. Soc. Chim. France 1977, 941-946; Cassani et al., Tetrahedron Lett. 1980, 1497-1500). Den Verfahren gemeinsam ist die Verwendung von schwierig handzuhabenden, empfindlichen und teuren Reagenzien, die eine Durchführung im technischen Maßstab nicht gestatten.

Daneben wurden Synthesewege beschrieben, die aufgrund hoher Stufenzahl unrentabel erscheinen (Roelofs et al., US. 3 845 108). In der französischen Offenlegungssschrift 2 505 820 wurde ein 4-stufiges Verfahren ausgehend von 9-Hydroxy-nonan-1-al beschrieben, wobei in der letzten Verfahrensstufe eine Kettenverlängerung durch Umsetzung von ggf. an der OH-Funktion acyliertem 9-Hydroxy-2-nonenal mit Phosphoryliden stattfindet.

Der Erfindung lag nun die Aufgabe zugrunde, ein kostengünstiges, im technischen Maßstab durchführbares Verfahren zur Herstellung von E7/Z9-Alka dienolen I, insbesondere solchen, die biologisch aktiv sind wie das E7/Z9-Dodecadienylacetat, bereitzustellen.

Demgemäß wurde ein Verfahren zur Herstellung von E7/Z9-Alkadien-1-olen der allgemeinen Formel I

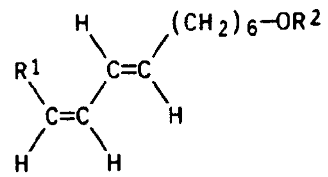

I,

in der

$R^1$ $C_1$-$C_8$-Alkyl und

$R^2$ Wasserstoff oder eine basenstabile Alkoholschutzgruppe bedeuten, gefunden, das

dadurch gekennzeichnet ist, daß man ein ggf. an der OH-Funktion geschütztes 7-Hydroxyheptan-1-al der Formel II

$$OHC\text{-}(CH_2)_6\text{-}OR^2$$

mit einem Phosphorylid der allgemeinen Formel III

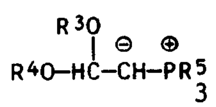

III,

in der

$R^3$, $R^4$ eine niedermolekulare Alkylgruppe bedeuten oder miteinander zu einem gegebenenfalls $C_1$-$C_4$-alkylsubstituierten Dioxan oder Dioxolansystem verbunden sind und

$R^5$ für Alkyl, Cycloalkyl oder ggf. substituiertes Phenyl steht,

im Sinne einer Wittig-Reaktion umsetzt zu Verbindungen der allgemeinen Formel IV

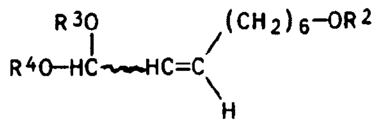

IV,

die man unter Einwirkung von Säure in das trans-konfigurierte 2-Nonen-al der Formel V

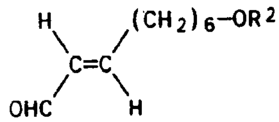

. V

überführt, das man durch eine weitere Wittig-Reaktion mit einem Phosphorylid der Formel VI

$$R^1-\overset{\ominus}{C}H-\overset{\oplus}{P}R^5_3 \quad VI,$$

worin $R^1$ und $R^5$ die obengenannte Bedeutung haben, zu den E7/Z9-Alkadienolen I umsetzt, die man in an sich bekannter Weise aus dem Reaktionsgemisch gewinnt.

Die vorliegende Erfindung beschreibt den Aufbau des Diensystems des gewünschten Pheromons durch zwei aufeinanderfolgende Wittig-Reaktionen. Das Verfahren zeichnet sich durch einen kurzen Reaktionsweg aus, bedient sich leicht zugänglicher, preiswerter und einfach handhabbarer Ausgangsmaterialien, erlaubt die Durchführung im technischen Maßstab und führt überwiegend zu dem gewünschten Stereoisomeren.

Die einzelnen Schritte des Verfahrens werden durch folgendes Reaktionsschema veranschaulicht:

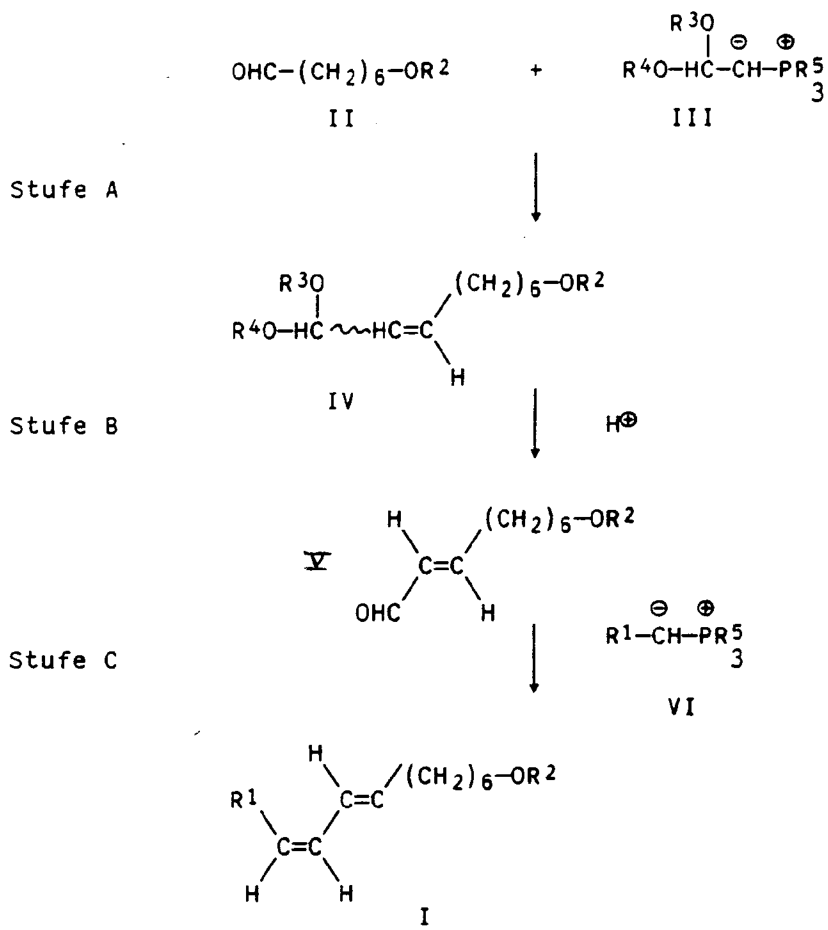


II III IV V VI I

Das 7-Hydroxyheptanal II bzw. dessen an der OH-Funktion geschützten Derivate sind an sich bekannt oder nach bekannten Methoden, z.B. wie in J. Chem. Ecol. 11 (1), 113 (1985) beschrieben, herstellbar.

Als Schutzgruppen $R^2$ kommen insbesondere basenstabile Gruppen wie $C_4$-$C_{12}$-tert-Alkylgruppen, die in 1-Position ein tertiäres Kohlenstoffatom tragen wie tert.-Butyl, 1,1-Dimethylprop-1-yl, 1,1-Dimethylbut-1-yl; $C_3$-$C_8$-Trialkylsilylgruppen wie Trimethylsilyl, Triethylsilyl, Tri-n-propylsilyl, Tri-isopropylsilyl, Tri-n-butylsilyl, Dimethylethylsilyl, Diethylmethylsilyl und Dimethyl-n-butylsilyl; Benzyl; Acyle, z.B. $C_2$-$C_4$-Alkanoyle wie Acetyl, Propionyl und Butyryl; Benzoyl; acyclische oder cyclische acetalische Gruppen wie $C_2$-$C_9$-Alkoxymethoxy, $C_3$-$C_{10}$-1-Alkoxyethoxy, 2-Furanyl, 2-Tetrahydrofuranyl, 2-Pyranyl, 2-Tetrahydropyranyl, 1,3-Dioxan-2-yl, 1,3-Dioxan-4-yl und 1,4-Dioxan-2-yl in Betracht. Da im Pheromon von Lobesia botrana die Acetylgruppe vorliegt, wird diese Schutzgruppe bevorzugt.

Die Reste $R^3$ und $R^4$ stehen für niedermolekulare Alkylgruppen, z.B. verzweigte oder unverzweigte $C_1$-$C_8$-, vorzugsweise $C_1$-$C_4$-Alkyl wie Methyl, Ethyl, Propyl, Isopropyl, n-Butyl, iso-Butyl, sec-Butyl, tert-Butyl, Pentyl, Hexyl oder Octyl. Beide Reste $R^3$ und $R^4$ können auch miteinander eine Ethylen- oder Propylenbrücke bilden, so daß ein Dioxan- bzw. Dioxolansystem vorliegt.

Die Reste $R^5$ stehen für $C_1$-$C_8$-Alkyl, $C_5$- oder $C_6$-Cycloalkyl und insbesondere ggf. substituiertes Phenyl, wobei als Substituenten inerte Gruppen wie $C_1$-$C_4$-Alkyl, $C_1$-$C_4$-Alkoxy oder Halogen wie Fluor, Chlor oder Brom in Betracht kommen. Bevorzugt sind unsubstituierte Phenylreste, da der für die Herstellung der Ylide verwendete Ausgangsstoff Triphenylphosphin besonders kostengünstig ist und zudem bei den Umsetzungen das sehr reaktionsträge und gut abtrennbare feste Triphenylphosphinoxid entsteht.

Die Phosphorylide III bzw. VI können in an sich bekannter Weise, z.B. wie in J. March, Advanced Organic Chemistry, 2. Aufl., S. 864-872, 1977, McGraw-Hill Kogakusha, Ltd. und der dort zitierten Literatur beschrieben durch Deprotonierung der entsprechenden Phosphoniumhalogenide, vorzugsweise -bromide oder -chloride, mittels einer Base hergestellt werden. Die Deprotonierung wird separat oder vorteilhaft in situ in einem aprotischen Lösungsmittel bei Raumtemperatur oder Temperaturen von 0 bis 100°C vorgenommen (vgl. J. Chem. Soc., Perkin I, 1974, 37-41). Als Lösungsmittel dienen z.B. am Stickstoff vollständig substituierte Säureamide wie Dimethylformamid, Dimethylacetamid, Diethylformamid oder N-Methylpyrrolidon; Sulfone wie Dimetylsulfon, Sulfoxide wie Dimethylsulfoxid; Ether wie Tetrahydrofuran; Glykolether, wie Dimethoxyethan, Diethoxyethan, Diglyme; aromatische Kohlenwasserstoffe wie Benzol oder Toluol, halogenierte Kohlenwasserstoffe wie Methylenchlorid oder Chlorbenzol. Es können auch wäßrig-organische Zweiphasensysteme wie $H_2O$/Toluol oder $H_2O$/Chlorbenzol verwendet werden.

Als Basen dienen vorzugsweise niedermolekulare Alkoholate, z.B. Alkalialkoholate wie Natriummethylat oder Natriumethylat sowie Alkalihydroxide, z.B. Natrium- oder Kaliumhydroxid bei Verwendung eines Zweiphasensystems. Daneben können Basen wie Butyllithium, Alkalihydride oder -amide verwendet werden, wobei die Reaktion ggf. unter Schutzgas durchgeführt wird.

Die Basenmenge ist nicht besonders kritisch, üblicherweise werden pro Mol Phosphoniumhalogenid stöchiometrische Mengen der Base oder ein geringer Überschuß, z.B. 1 bis 20 Mol.% Überschuß verwendet. Größere Mengen sind möglich, bringen in der Regel aber keine weiteren Vorteile.

Pro Mol der für die Wittig-Reaktion eingesetzten Aldehyde II bzw. V können stöchiometrische Mengen oder vorteilhaft ein geringer Überschuß, z.B. 10 bis 20 Mol.% Überschuß der Ylide III bzw. VI verwendet werden. Höhere oder auch geringere Mengen sind möglich.

Das nach der ersten Wittig-Reaktion gebildete $\alpha,\beta$-ungesättigte Acetal IV fällt als cis/trans-Gemisch an und wird bei der nachfolgenden Spaltung des Acetals durch saure Hydrolyse in den einheitlich trans-konfigurierten Aldehyd V überführt.

Als Säuren können Mineralsäuren oder organische Säuren wie Carbonsäuren, Dicarbonsäuren oder Sulfonsäuren verwendet werden. Beispielsweise seien Salzsäure, Essigsäure, Oxalsäure oder Toluolsulfonsäure genannt. Pro Mol Acetal IV werden üblicherweise 1 bis 2 Säureäquivalente verwendet. Vorteilhaft wird die Säurebehandlung mit verdünnter Salzsäure, z.B. 5 bis 30 %iger Salzsäure bei Temperaturen von 0 bis 50°C in einem inerten Lösungsmittel, z.B. einem Ether wie Diethylether, Methyl-tert-butylether oder Tetrahydrofuran durchgeführt.

Bei der nachfolgenden zweiten Wittig-Reaktion, die an sich aus der Literatur z.B. der französischen Offenlegungsschrift 2 505 820 bekannt ist, fällt das gewünschte Dien I mit einem EZ/EE-Verhältniss von ca. 85:15 an und kann in an sich bekannter Weise isoliert und z.B. durch Säulenchromatographie gereinigt werden.

Ein Vorteil des erfindungsgemäßen Verfahrens liegt in der einfachen Durchführbarkeit. So können die anfallenden Zwischenprodukte ohne aufwendige Reinigungschritte für die Folgestufen eingesetzt werden. Das bei den Wittig-Reaktionen gebildete Phosphinoxid braucht erst am Ende der Reaktionssequenz abgetrennt zu werden.

Die nachfolgenden Beispiele erläutern das Verfahren.

Beispiel 1

Herstellung der trans-2-Nonenale V

172 g (1,0 mol) 7-Acetoxyheptanal und 514 g (1,2 mol) (1,3-Dioxolan-2-yl)-methyl-triphenylphosphoniumbromid, werden in 3 l Dimethylformamid auf 90°C aufgeheizt und portionsweise mit 65 g (1,2 mol) Natriummethylat versetzt. Nach 6-stündigem Nachrühren bei 90°C und Abkühlen erfolgt die Aufarbeitung durch Zugabe von 6 l Wasser und Extraktion mit Methyl-tert.-butylether. Nach Waschen und Trocknen erhält man 243 g Produktgemisch, das in 300 ml Tetrahydrofuran aufgenommen und mit 300 ml 10 %iger Salzsäure versetzt wird. Nach 3 h Rühren bei Raumtemperatur und Zugabe von 500 ml Wasser wird mit Methyl-tert.-butylether extrahiert und die organische Phase gewaschen und getrocknet.

Man erhält 187 g Produktgemisch, das nach gaschromatographischer Analyse aus 47 % 9-Hydroxy-2-nonenal sowie aus 18 % 9-Acetoxy-2-nonenal besteht; dies entspricht einer Gesamtausbeute von 73 %. Das Rohprodukt kann für die folgenden Umsetzungen, d.h. Wittig-Reaktion und ggf. Veresterung der freien OH-Gruppe direkt eingesetzt werden.

Beispiel 2

Umsetzung von 9-Acetoxy-2-nonenal zu E7/Z9-Dodecadienylacetat

385 g (1,00 mol) Propyltriphenylphosphoniumbromid werden in 3 l Tetrahydrofuran vorgelegt und portionsweise mit 112 g (1 mol) Kalium-tert.-butylat versetzt. Nach 30 min Rühren bei Raumtemperatur werden 217 g (0,835 mol) 9-Acetoxy-2-nonenal (76 %ig, Rohprodukt) zugetropft und 45 min bei Raumtemperatur gerührt. Durch Zugabe von 500 ml $H_2O$ wird die Reaktion abgebrochen. Die Aufarbeitung erfolgt durch Zugabe von NaCl-Lösung, Extraktion mit Diethylether und Trocknen über $Na_2SO_4$; anschließend wird das Rohprodukt in Essigester/Petrolether (1:1) über Kieselgel abfiltriert. Man erhät 242 g Produkt, das nach gaschromatographischer Analyse zu 24 % aus E7/Z9-Dodecadienol sowie 44 % E7/Z9-Dodecadienylacetat besteht.

Zu diesem Gemisch werden 60 ml (0,63 mol) Essigsäureanhydrid und 65 ml (0,80 mol) Pyridin zugegeben und 3h unter Rückfluß erhitzt. Zugabe von Eiswasser, Extraktion mit Methylenchlorid, Waschen der organischen Phase mit 1 %iger Salzsäure und Wasser sowie Trocknen über $Na_2SO_4$ liefert 259 g Produktgemisch, das nach gaschromatographischer Analyse 69,2 % E7/Z9-Dodecadienylacetat enthält; dies entspricht einer Gesamtausbeute von 96 %.

Eine weitere Reinigung erfolgt durch Destillation.

## Patentansprüche

1. Verfahren zur Herstellung von E7/Z9-Alkadien-1-olen der allgemeinen Formel I

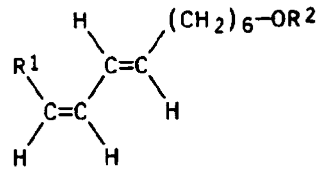

I,

in der
$R^1$ $C_1$-$C_8$-Alkyl und
$R^2$ Wasserstoff oder eine basenstabile Alkoholschutzgruppe bedeuten,
dadurch gekennzeichnet, daß man ein ggf. an der OH-Funktion geschütztes 7-Hydroxyheptan-1-al der Formel II

$$OHC\text{-}(CH_2)_6\text{-}OR^2 \qquad II$$

mit einem Phosphorylid der allgemeinen Formel III

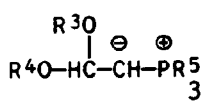

III,

in der
$R^3$, $R^4$ eine niedermolekulare Alkylgruppe bedeuten oder miteinander zu einem gegebenenfalls $C_1$-$C_4$-alkylsubstituierten Dioxan- oder Dioxolansystem verbunden sind und
$R^5$ für Alkyl, Cycloalkyl oder ggf. substituiertes Phenyl steht,
im Sinne einer Wittig-Reaktion umsetzt zu Verbindungen der allgemeinen Formel IV

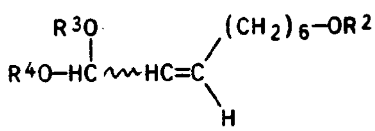

IV,

die man unter Einwirkung von Säure in das trans-konfigurierte 2-Nonen-al der Formel V

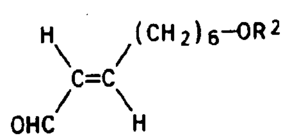

V

überführt, das man durch eine weitere Wittig-Reaktion mit einem Phosphorylid der Formel VI

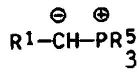

VI,

worin $R^1$ und $R^5$ die obengenannte Bedeutung haben, zu den E7/Z9-Alkadienolen I umsetzt, die man in an sich bekannter Weise aus dem Reaktionsgemisch gewinnt.

2. Verfahren nach Anspruch 1, dadurch gekennzeichnet, daß man als Alkoholschutzgruppe $R^2$ Acetyl verwendet.

3. Verfahren nach Anspruch 1, dadurch gekennzeichnet, daß die Reste $R^5$ im Phosphorylid III Phenylreste darstellen.

4. Verfahren nach Anspruch 1, dadurch gekennzeichnet, daß man die Wittig-Reaktionen in einem aprotischen Lösungsmittel durchführt.

5. Verfahren nach Anspruch 1, dadurch gekennzeichnet, daß man als Säure verdünnte Salzsäure verwendet.

6. Verfahren nach Anspruch 1, dadurch gekennzeichnet, daß man die Säure bei Temperaturen von 0 bis 50°C auf das Acetal IV einwirken läßt.

7. Verfahren nach Anspruch 1, dadurch gekennzeichnet, daß man pro Mol Acetal IV 1 bis 2 Säureäquivalente verwendet.

## Claims

1. A process for the preparation of an E7/Z9-alkadien-1-ol of the formula I

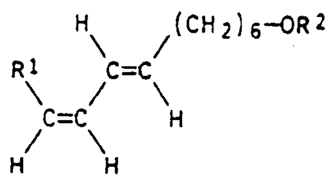

I,

where $R^1$ is $C_1$-$C_8$-alkyl and $R^2$ is hydrogen or a base-stable alcohol protective group, wherein a 7-hydroxyheptan-1-al, which may be protected at the OH function, of the formula II

$$OHC\text{-}(CH_2)_6\text{-}OR^2 \qquad II$$

is subjected to a Wittig reaction with a phosphorylide of the formula III

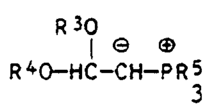

III,

where $R^3$ and $R^4$ are each a low molecular weight alkyl group or are bonded to one another to form an unsubstituted or $C_1$-$C_4$-alkyl-substituted dioxan or dioxolan system and $R^5$ is alkyl, cycloalkyl, phenyl or substituted phenyl, to give a compound of the formula IV

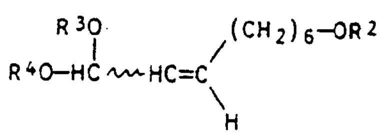

IV,

which is converted by the action of an acid into a trans-2-nonenal of the formula V

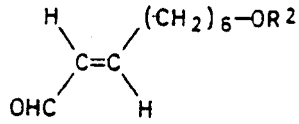

V

which is converted by a further Wittig reaction with a phosphorylide of the formula VI

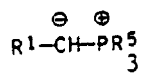

VI

where $R^1$ and $R^2$ have the abovementioned meanings, to an E7/Z9-alkadienol I, which is obtained from the reaction mixture in a conventional manner.

**2.** A process as claimed in claim 1, wherein the alcohol protective group $R^2$ used is acetyl.

**3.** A process as claimed in claim 1, wherein $R^5$ in the phosphorylide III is phenyl.

**4.** A process as claimed in claim 1, wherein the Wittig reactions are carried out in an aprotic solvent.

**5.** A process as claimed in claim 1, wherein the acid used is dilute hydrochloric acid.

**6.** A process as claimed in claim 1, wherein the acid is allowed to act on the acetal IV at from 0 to 50°C.

**7.** A process as claimed in claim 1, wherein from 1 to 2 acid equivalents are used per mole of acetal IV.

## Revendications

**1.** Procédé de préparation de E7/Z9-alcadiène-1-ols de formule générale

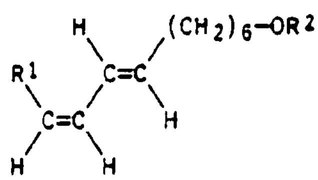

I,

dans laquelle
$R^1$ représente un groupe alkyle en C 1-C 8, et
$R^2$ représente l'hydrogène ou un groupe protecteur de la fonction alcool stable aux bases, caractérisé en ce que l'on fait réagir le 7-hydroxyheptane-1-al éventuellement protégé sur la fonction OH, de formule II

$OHC\text{-}(CH_2)_6\text{-}OR^2$ II

avec un phosphorylide de formule générale III

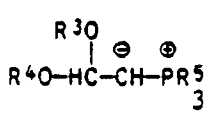

III,

dans laquelle
$R^3$, $R^4$ représentent chacun un groupe alkyle inférieur ou forment ensemble un système cyclique dioxanne éventuellement substitué par des groupes alkyle en C 1-C 4, et
$R^5$ représente un groupe alkyle, cycloalyle ou phényle éventuellement substitué, dans le sens d'une réaction de Wittig, ce qui donne des composés de formule générale IV

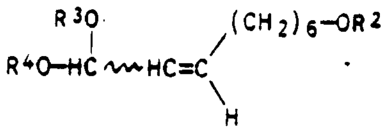 IV,

qu'on convertit, sous l'action d'un acide, en le 2-nonène-al en configuration trans de formule V

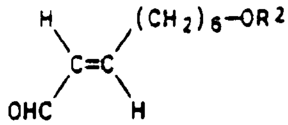 V

qu'on convertit lui-même, par une autre réaction de Wittig avec un phosphorylide de formule VI

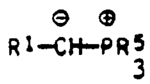 VI,

dans laquelle $R^1$ et $R^5$ ont les significations indiquées ci-dessus, en les E7/Z9-alcadiène-1-ols I, lesquels sont isolés de manière connue en soi du mélange de réaction.

2. Procédé selon la revendication 1, caractérisé en ce que le groupe protecteur de la fonction alcool $R^2$ est un groupe acétyle.

3. Procédé selon la revendication 1, caractérisé en ce que les symboles $R^5$ du phosphorylide III représentant des radicaux phényle.

4. Procédé selon la revendication 1, caractérisé en ce que l'on effectue les réactions de Wittig dans un solvant aprotique.

5. Procédé selon la revendication 1, caractérisé en ce que l'acide utilisé est l'acide chlorhydrique dilué.

6. Procédé selon la revendication 1, caractérisé en ce que l'on fait agir l'acide sur l'acétal IV à des températures de 0 à 50 degrés C.

7. Procédé selon la revendication 1, caractérisé en ce que l'on utilise l'acide en quantité de 1 à 2 équivalents par mol de l'acétal IV.